# FASCICULE DE BREVET EUROPEEN

(11) **EP 1 648 350 B1**
(45) Date de publication et mention de la délivrance du brevet: **15.04.2009**
(21) Numéro de dépôt: 04767819.8
(22) Date de dépôt: 29.07.2004
(51) Int. Cl.: A61F 2/34

(54) **Implant acétabulaire et procédé de fabrication de cet implant**
Hüftpfannenimplantat und Verfahren zur Herstellung dieses Implantats
Acetabular implant and method for manufacturing said implant

(30) Priorité: 30.07.2003 FR 0309405
(43) Date de publication de la demande: 26.04.2006
(73) Titulaire: Depuy (Ireland) Limited, County Cork, Ringaskiddy (IE); Balay, Bruno, 01600 Trevoux (FR); Cartillier, Jean-Claude, 69008 Lyon (FR); Charlet, Claude, 69370 Saint Didier au Mont d'Or (FR); Chatelet, Jean-Christophe, 01150 Chazey Sur Ain (FR); Fessy, Michel-Henri, 69390 Charly (FR); Hovy, Louis, 64367 Mühltal-Traisa (DE); Machenaud, Alain, 74330 La Balme de Sillingy (FR); Semay, Jean-Marc, 42270 Saint Priest en Jarez (FR); Setiey, Louis, 69400 Gleize (FR); Vidalain, Jean-Pierre, 74940 Annecy le Vieux (FR); Witzel, Ulrich, 42279 Wuppertal (DE); Zanello, Sylvain, 69780 Mions (FR)
(72) Inventeur: BALAY, Bruno, F-01600 Trevoux (FR); CARTILLIER, Jean-Claude, F-69008 Lyon (FR); CHARLET, Claude, F-69370 Saint Didier au Mont D'or (FR); CHATELET, Jean-Christophe, 01480 Jassans Riottier (FR); FESSY, Michel-Henri, 69230 ST GENIS LAVAL (FR); HOVY, Louis, 64367 Mühltal-Traisa (DE); MACHENAUD, Alain, F-74330 La Balme de Sillingy (FR); SEMAY, Jean-Marc, F-42270 Saint Priest en Jarez (FR); SETIEY, Louis, F-69400 Gleize (FR); VIDALAIN, Jean-Pierre, F-74940 Annecy le Vieux (FR); WITZEL, Ulrich, 42279 Wuppertal (DE); ZANELLO, Sylvain, F-69780 Mions (FR)
(74) Mandataire: Lagrange, Jacques Etienne M.M.
(86) Numéro de dépôt international: PCT/FR2004/002045
(87) Numéro de publication internationale: WO 2005/011537

(56) Documents cités:
- EP-A- 0 578 345
- EP-A- 0 887 052
- EP-A- 1 151 732
- WO-A-01/45585
- WO-A-95/17140
- DE-A- 4 211 343
- DE-C- 3 535 959
- DE-C- 4 336 551
- FR-A- 2 688 402
- FR-A- 2 748 201
- US-A- 4 883 491
- US-A- 5 505 736

## Description

La présente invention a trait à un implant acétabulaire, c'est-à-dire au composant destiné à être implanté dans le cotyle, dans le cadre d'une prothèse de hanche. L'invention a également trait à un procédé de fabrication de cet implant.

Les implants acétabulaires comportent généralement une cupule destinée à être introduite et fixée dans le cotyle de l'articulation de la hanche et un insert, fixe ou mobile dans la cupule, formant le siège de l'articulation de la tête prothétique fémorale. Les cupules, généralement réalisées en un métal biocompatible tel que le titane, peuvent être fixées dans la cavité cotyloïdienne en utilisant des ciments. Lorsque l'on opte pour une fixation sans ciment, il est nécessaire d'assurer une fixation primaire de la cupule dans la cavité cotyloïdienne, cette fixation étant éventuellement complétée, notamment en cas de reprise de la prothèse, par des moyens complémentaires tels que des vis, crochets ou pattes obturatrices.

On doit, cependant, obtenir ensuite une fixation secondaire par ostéointégration, grâce à une ostéogénèse de cicatrisation, qui va pérenniser la stabilité de l'implant dans le site osseux du cotyle en remodelage sous contrainte permanente.

La fixation mécanique primaire peut être obtenue soit par impaction, en utilisant, au besoin, des cupules avec effet de surface pour obtenir un ajustement par pression, soit par vissage, les cupules étant alors pourvues de moyens tels que des filets permettant un vissage dans l'os cotyloïdien. On cherche à faciliter cette ostéointégration soit en rendant la surface de la cupule rugueuse, par exemple, par sablage ou corindonnage, soit en recouvrant la cupule d'un revêtement réhabitable à effet ostéointégrateur, qui est généralement une céramique d'hydroxyapatite de calcium.

Un implant acétabulaire reflétant l'art antérieur est décrit dans US-A-4 883 491. La présente invention se propose de perfectionner les cupules du type à visser et revêtues d'un matériau d'ostéointégration, tel qu'une céramique d'hydroxyapatite.

Elle permet notamment, de faciliter la fixation primaire par vissage et ajustage de ces cupules et d'améliorer, après stabilisation initiale, la fixation secondaire par ostéointégration, véritable ostéogénèse de cicatrisation qui va pérenniser la stabilité de l'implant dans un site osseux en remodelage permanent.

Elle permet également de diminuer l'importance du traumatisme osseux lors de la fixation par vissage.

Enfin elle permet de diminuer ou supprimer les risques de formation d'éclats à partir du revêtement.

L'invention a pour objet un implant cotyloïdien du type comprenant une cupule à visser recevant un insert articulaire, ladite cupule présentant à sa périphérie, et notamment dans sa zone tropico-équatoriale des moyens de vissage destinés à pénétrer dans la matière osseuse du cotyle lors du vissage, ladite cupule portant un revêtement facilitant l'ostéointégration, tel que, notamment, un revêtement d'hydroxyapatite de calcium sélectif caractérisé en ce que le revêtement est de type épais sur les parties convexes de la surface externe de la cupule, y compris dans les zones ou vallées ou creux de filets laissés libres dans les moyens de vissage, tandis que ce revêtement présente une épaisseur inférieure, ou est même absent, sur les reliefs ou filets de vissage.

Par revêtement de type épais, on entend une épaisseur habituelle de revêtement telle qu'elle est généralement connue sur les cupules revêtues ou sur d'autres prothèses revêtues d'une couche de revêtement d'ostéointégration. Dans le cas de l'hydroxyapatite de calcium, cette épaisseur de revêtement de type épais est de préférence de l'ordre de 100 à 200 microns, notamment avantageusement de l'ordre de 150 ± 35 microns, ces indications n'étant pas limitatives et dépendant de la nature et de la qualité du revêtement ostéointégrateur.

Au sens de l'invention, on comprend que cette épaisseur peut atteindre toute la valeur permise dans une position d'implantation intra-osseuse stable, essentiellement soumise à des efforts de compression.

Les reliefs de vissages, tels que les filets, peuvent alors, soit ne présenter aucun revêtement, soit, de préférence, présenter un revêtement moins épais, qui peut être avantageusement, dans le cas de l'hydroxyapatite, de l'ordre de 50 ± 30 microns.

On comprend que l'on obtiendra ainsi des qualités d'ostéointégration importantes au niveau de la couche épaisse de revêtement sur les parties convexes de la cupule, y compris entre les filets, la couche de revêtement n'étant guère soumise à des forces de cisaillement durant le vissage de la cupule et le contact s'établissant sans traumatisme en fin de vissage, au moment du blocage dans la cavité cotyloïdienne.

Sur les flancs de filets ou de reliefs, la moindre épaisseur du revêtement améliore la résistance au cisaillement de ce revêtement et diminue donc les risques de délamination lors du vissage, tout en assurant un excellent accrochage mécanique et en permettant, également, une ostéointégration améliorée par la présence de l'hydroxyapatite.

Dans le cas où l'hydroxyapatite n'est pas présente sur les filets, les flancs de filets sont avantageusement rendus rugueux par sablage ou corindonnage, améliorant également l'ostéogénèse de contact.

Dans une forme de réalisation particulièrement préférée de l'invention, le moyen de vissage, tels que des filets, est agencé pour traumatiser le moins possible la zone cotyloïdienne dans laquelle pénètrent les filets, et pour présenter un maximum de surface convexe, c'est-à-dire de vallées entre les flancs de filets pour faciliter, à ce niveau, l'ostéointégration par ostéogénèse de contact et remodelage sous contrainte.

Conformément à cette forme de réalisation perfectionnée, le relief de vissage est agencé pour exercer un effet de coupe autotaraudant lors du vissage et un effet de compression de l'os spongieux.

De façon particulièrement préférée, les filets, qui sont disposés sur la zone tropico-équatoriale de la cupule, ont une section étroite pour laisser une surface maximale entre deux crêtes de filets consécutives, le rapport de l'épaisseur d'un filet au pas correspondant étant de préférence compris entre 0,2 et 0,5.

De préférence, la section des filets est asymétrique dans un plan diamétral, avec un angle plus faible, par exemple de l'ordre de 5 à 10° du côté polaire du filet, c'est-à-dire un filet proche de l'horizontale, pour s'opposer à l'enfoncement et un angle supérieur, par exemple de l'ordre de 15 à 20° du côté équatorial, afin d'assurer un bon effet de compression, lors de la mise en contrainte de l'os recevant le filetage.

De façon avantageuse également, les crêtes de filets sont détalonnées, avec un bord d'attaque radialement plus élevé que le reste de la crête, dont la hauteur radiale décroît de préférence progressivement vers l'arrière du filet.

De préférence, le bord d'attaque est lui-même incliné en étant formé par une passe de fraisage fortement inclinée en biais par rapport à l'inclinaison du filetage lui-même, l'arête d'attaque étant de préférence elle-même orientée agressivement vers l'avant par rapport à la radiale.

Le pas du filetage est avantageusement régulier afin de créer un seul sillon osseux, dans lequel pénètrent des filets successifs lors de la rotation de vissage.

De préférence la hauteur des filets, ou plus précisément l'allure des crêtes, dans toute la zone, est constante, étant entendu que lorsqu'il s'agira d'une cupule de reprise, cette hauteur sera plus importante pour permettre un vissage dans une cavité cotyloïdienne plus irrégulière avec une qualité osseuse moindre.

De préférence également, la cupule présente une convexité parfaitement sphérique, y compris entre les reliefs successifs, permettant ainsi une excellente adaptation contre le fond de cavité cotyloïdienne obtenu après fraisage, lorsque la cupule parvient en fin de vissage.

Dans une forme de réalisation très avantageuse de l'invention, on peut utiliser, par exemple, une forme de filetage telle que celle décrite dans les demandes de brevets EP-A-0887052 et DE-A-19727846, notamment sur les figures 9 à 14 de EP 98250211, le dessin et la description de cette demande étant ici incorporés par référence.

L'invention a également pour objet un procédé de fabrication des cupules d'implant selon l'invention dans lequel on réalise une cupule, par exemple en alliage de titane, ayant un filetage dans sa zone tropico-équatoriale, on effectue un traitement de la surface externe de la cupule pour la rendre rugueuse, par exemple par sablage ou corindonnage, et l'on met en place un revêtement de matériau ostéointégrateur tel que, et notamment, de l'hydroxyapatite de calcium, sur ladite surface de cupule, caractérisé en ce que l'on mène l'étape de revêtement de matériau ostéointégrateur, de façon à réaliser un dépôt épais sur les surfaces convexes de la cupule, y compris les vallées séparant les filets voisins et en diminuant ou supprimant le revêtement par ledit matériau sur les flancs et bords des filets.

Dans le cas habituel où l'on utilise une torche du type à plasma pour projeter et fixer sur la surface rugueuse de la cupule le matériau destiné à former ledit revêtement, on peut avantageusement réduire l'épaisseur du dépôt formant le revêtement sur les filets par modification temporaire de l'angle d'inclinaison de la torche et/ou par modification de la vitesse de défilement relative entre la torche et la cupule.

Dans un mode de mise en oeuvre préféré, l'invention a pour objet un procédé de production de cupules d'implant selon l'invention caractérisé par les étapes suivantes :
- on détermine à l'avance, avec éventuellement modification pendant le processus de production, le nombre de cupules revêtues ou non d'hydroxyapatite de calcium à fabriquer ;
- on réalise les cupules non revêtues et on les soumet à un traitement destiné à rendre leur surface rugueuse ;
- on détermine dans la production d'ensemble ainsi déterminée la proportion de cupules destinées à recevoir un revêtement de matériau ostéointégrateur, tel que de l'hydroxyapatite de calcium ;
- on procède au revêtement de cette cupule conformément à l'invention ;
- on conditionne et on stérilise l'ensemble des cupules pour être contenues dans des emballages stériles individuels, lesdits emballages présentant des marquages différents pour distinguer les cupules revêtues des cupules non revêtues.

La détermination du nombre total de cupules à fabriquer est effectuée d'une façon classique, soit par prévision, soit par centralisation des commandes ou les deux.

La détermination de la proportion de cupules à revêtir par rapport aux cupules qui ne sont pas revêtues, est de préférence faite pendant le processus de production et éventuellement ajustée en fonction de cette évolution.

Ces opérations peuvent être effectuées en utilisant les moyens informatiques, pouvant, éventuellement, intervenir dans le pilotage des moyens de production.

D'autres avantages et caractéristiques de l'invention apparaîtront à la lecture de la description suivante, faite à titre d'exemple non limitatif et se référant au dessin annexé dans lequel :
la figure 1 représente une vue en perspective d'une cupule d'implant selon l'invention,
la figure 2 représente une vue en élévation de cette cupule,

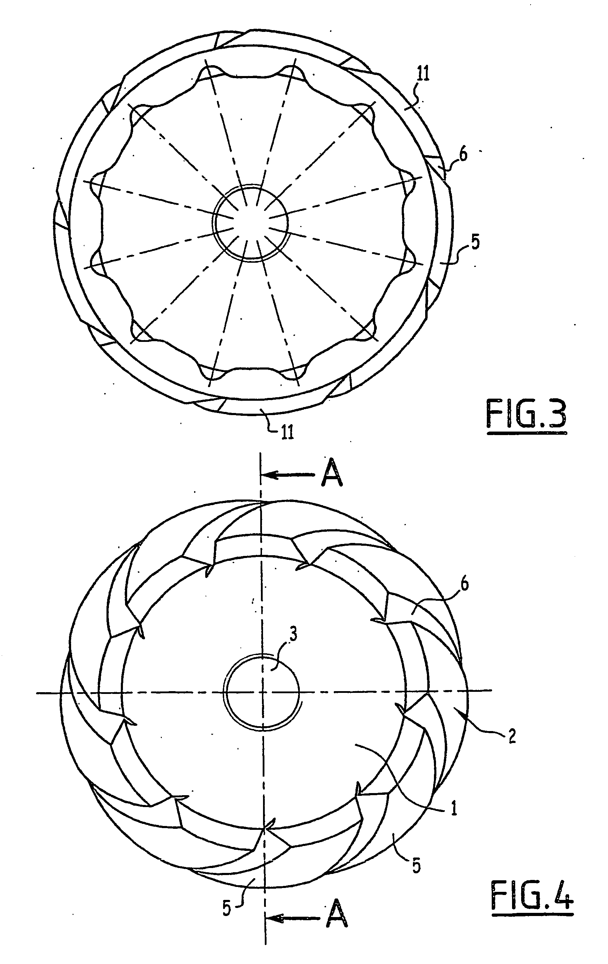

la figure 3 représente une vue de dessous de cette cupule,
la figure 4 représente une vue de dessus de la cupule,
la figure 5 représente une vue en coupe axiale de la cupule,
la figure 6 représente une section selon la coupe B-B de la figure 2,
la figure 7 représente une vue en coupe radiale, de la figure 5, d'un détail avec agrandissement au niveau du filetage,
la figure 8 représente une vue schématique illustrant les zones ayant un revêtement épais et les zones ayant un revêtement mince d'hydroxyapatite de calcium.

La cupule représentée présente généralement une forme de calotte hémisphérique avec une surface extérieure polaire 1 sans relief et une zone extérieure inter tropico-équatoriale 2 présentant le filetage. Un trou polaire 3, généralement utilisé pour les ancillaires de pose, est présent au pôle même de la cupule.

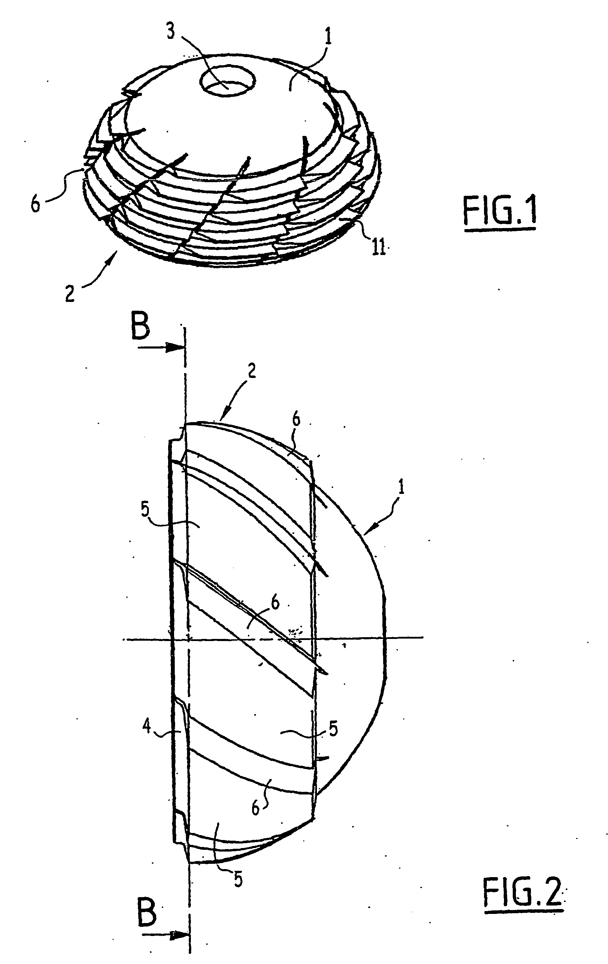

En se référant tout particulièrement à la figure 2, on voit que la zone équatoriale portant les moyens de vissage s'étend à peu de distance de la base 4 de la cupule et que cette zone équatoriale est elle-même divisée en une pluralité de segments de zone 5 par des fraisages hélicoïdaux sur sphère 6 destinés à former les bords d'attaque autotaraudants des filets du filetage de la zone 2.

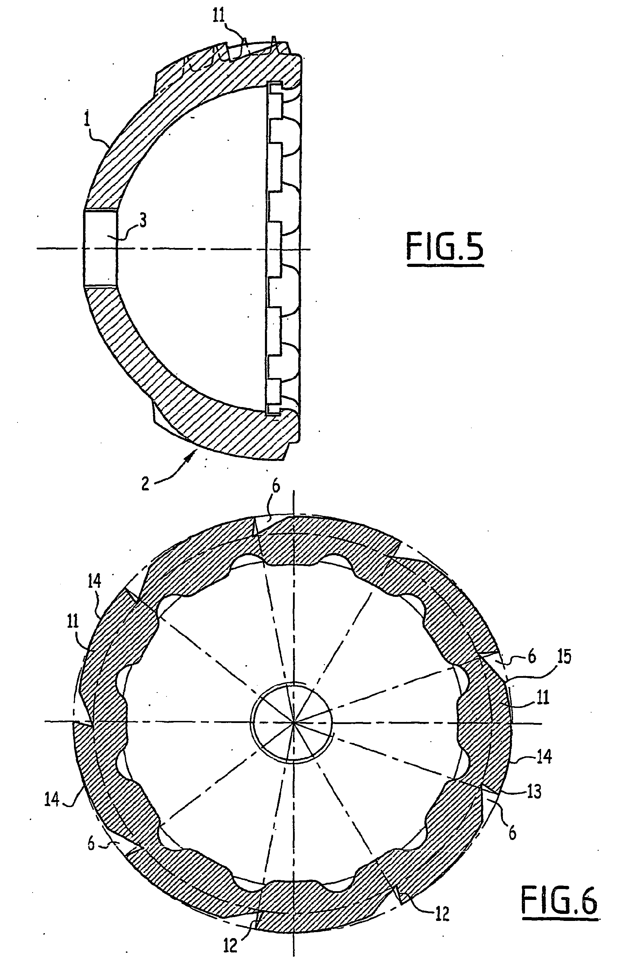

Sur la plupart des figures, les filets individuels ne sont pas représentés, mais on les distingue bien sur les figures 1, 5, 7, ainsi que pour les filets les plus équatoriaux, sur la figure 6.

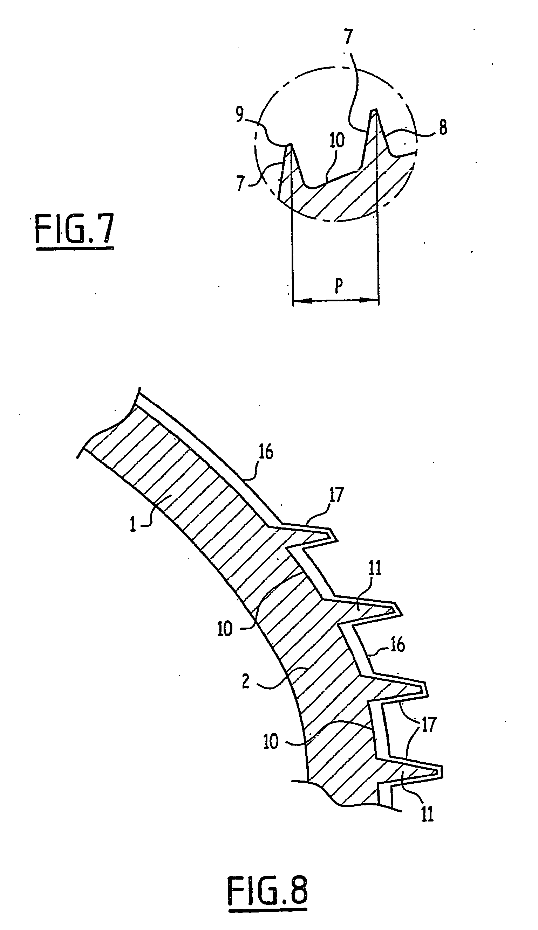

Dans l'exemple représenté, le filetage forme une seule hélice sphérique, c'est-à-dire une hélice déformée à la surface de la sphère de la cupule, et ayant un pas constant, P, défini sur l'axe de révolution, passant par le pôle de la cupule. Les filets ont eux-mêmes une inclinaison constante par rapport à cet axe. Plus précisément, comme on le voit sur la figure 7, les flancs polaires 7 ont une inclinaison, plus faible que celle des flancs équatoriaux 8, l'inclinaison des flancs polaires étant de 5 à 10° et celle des flancs équatoriaux de 15 à 20°, par rapport au plan radial perpendiculaire à l'axe polaire. Les sommets ou crêtes de filets sont légèrement chanfreinés. On voit, enfin, que les gorges ou fond de filets 10 présentent une largeur supérieure à celle de la base des filets, afin de fournir un maximum de surface convexe sphérique entre les filets.

La forme des filets individuels 11 sera mieux vue sur la figure 6. Chaque filet 11 s'étend entre deux fraisages successifs 6 et présente, notamment, les caractéristiques suivantes.

La dent d'attaque est déterminée par la gorge fraisée 6, le fraisage étant tel que l'arête de la dent s'étende vers l'intérieur et vers l'arrière pour former une arête de coupe inclinée avec une pointe agressive 13, l'arête formant avec la tangente au point 13 un angle inférieur à 90°. On comprend, par ailleurs, que, le passage de fraisage 6 étant incliné, comme on le voit sur les figures 1 et 2, la facette de coupe de la dent, qui s'étend vers le haut en direction polaire à partir de l'arête 12, regarde en direction polaire pour former une facette de coupe située dans un plan qui n'est pas perpendiculaire au plan de la figure 6.

La hauteur radiale de la crête 14 du filet 11 diminue progressivement depuis le sommet 13 jusque vers la fin 15 de l'arête, comme on le voit bien sur la figure 6, de sorte que la crête 14 forme une dépouille progressivement en retrait par rapport au fond de sillon taraudé par la pointe 13 dans l'os cotyloïdien.

Grâce à ces caractéristiques, on peut, à la fois, obtenir une excellente coupe franche déterminant les sillons et réduire les forces de friction pendant le vissage autotaraudant, alors que la poursuite du vissage, grâce aux inclinaisons des flancs de filets assure l'effort de compression souhaitable sur l'os pris entre les filets.

Une fois que la cupule a été fabriquée et les filets usinés selon la caractéristique précitée, la cupule est soumise à un traitement de surface par corindonnage, la totalité de la surface de la cupule, y compris les filets, acquerrant ainsi le degré de rugosité souhaitable, qui peut être défini de la façon suivante RT 25 microns mini.

On procède ensuite à la projection, à l'aide d'une torche à plasma, d'une poudre d'hydroxyapatite de calcium sur la surface externe de la cupule. La formation d'un revêtement d'hydroxyapatite de calcium par ce moyen est bien connue dans l'art et n'a pas besoin d'être décrite davantage en détail. Par exemple, on utilisera une torche de type torche à plasma située à 100 mm de la surface, avec un débit de projection d'hydroxyapatite de calcium de 10g/min et une vitesse de déplacement relative de la torche par rapport à la surface de 30 mm/s. Par exemple la torche suit une inclinaison variable et optimisée.

Conformément à l'invention, on réalise sur la totalité de la surface sphérique de la cupule, à savoir non seulement la zone polaire 1, mais également la zone équatoriale filetée 2, c'est-à-dire les vallées ou fond de filets 10, un revêtement épais d'hydroxyapatite ayant une épaisseur sensiblement constante qui est avantageusement de 150 microns. On a représenté sur la figure 8, par la ligne 16, l'image schématique de ce revêtement, cette image étant dans sa partie sphérique décalée de façon homothétique par rapport à la surface de la partie sphérique de la cupule. L'opération de projection d'hydroxyapatite est, par contre, menée au niveau des filets 11, de façon à ne produire qu'un revêtement mince de 50 microns, représenté par le trait 17, également décalé par rapport à la surface réelle de la cupule. On voit notamment que ce revêtement mince recouvre, à la fois les faces équatoriales et polaires et les crêtes des filets 11.

Par exemple, l'obtention de ce revêtement plus mince peut être obtenu en agissant sur les paramètres décrits précédemment.

On peut, éventuellement, grâce à l'inclinaison des torches, ne réaliser qu'un revêtement très mince ou même pratiquement nul de la facette de coupe de filet qui s'étend à partir du bord antérieur de coupe 12.

On peut ainsi, grâce à l'invention, réaliser des revêtements d'hydroxyapatite de calcium très épais sur les zones convexes sphériques, qui ne viennent interagir mécaniquement avec l'os spongieux sous-chondral, dégagé par le fraisage hémisphérique de la cavité cotyloïdienne, que tout à fait vers la fin du vissage, alors que, grâce à la faible épaisseur d'hydroxyapatite sur les filets eux-mêmes, on réduit notablement le couple de vissage et, par là également, la dégradation de l'os sous-chondral au contact direct des filets, cette préservation étant encore accrue par la forme aménagée des filets qui sont minces et présentent une arête supérieure détalonnée ainsi qu'un bord de coupe extrêmement tranchant.

On réduit également le risque de détachement ou de laminage du revêtement d'hydroxyapatite, notamment au niveau des dents, où la couche est soumise à l'effort le plus important, la couche mince étant beaucoup plus cohérente adhérant mieux à la surface corindonnée métallique sous-jacente.

La cupule selon l'invention, une fois posée, peut recevoir tout type d'insert articulaire, par exemple en polyéthylène, en alumine ou en métal.

Lors du procédé de production d'une pluralité de cupules selon l'invention, on peut avantageusement prévoir de fabriquer en même temps les cupules destinées à recevoir le revêtement selon l'invention et des cupules non revêtues, selon les besoins et ajuster si l'on veut en permanence la proportion entre cupules revêtues et cupules non revêtues, en orientant vers le poste de revêtement d'hydroxyapatite un nombre plus ou moins grand de cupules, les conditionnements stériles étant ensuite pilotés en fonction de cette proportion.

## Revendications

1. Implant cotyloïdien comprenant une cupule à visser recevant un insert articulaire, ladite cupule présentant à sa périphérie, et notamment dans sa zone tropico-équatoriale (2) des moyens de vissage destinés à pénétrer dans la matière osseuse du cotyle lors du vissage, ladite cupule portant un revêtement facilitant l'ostéointégration, **caractérisé en ce que** le revêtement est épais sur les parties convexes (1, 10) de la surface externe de la cupule, y compris dans les zones ou vallées ou creux de filets (10), laissés libres dans les moyens de vissage, tandis que ce revêtement présente une épaisseur inférieure, ou est même absent, sur les reliefs ou filets de vissage (11).

2. Implant selon la revendication 1 **caractérisé en ce que** l'épaisseur du revêtement épais est de 100 à 200 microns.

3. Implant selon la revendication 2 **caractérisé en ce que** l'épaisseur de revêtement est de l'ordre de 150 ± 35 microns.

4. Implant selon l'une des revendications 1 à 3 **caractérisé en ce que** les reliefs de vissages présentent un revêtement de l'ordre de 50 ± 30 microns.

5. Implant selon l'une des revendications 1 à 3, **caractérisé en ce que** les reliefs de vissage (11) ne présentent pas de revêtement et ont une surface rugueuse.

6. Implant selon l'une des revendications 1 à 5 **caractérisé en ce que** le moyen de vissage, agencé pour traumatiser le moins possible le site osseux cotyloïdien dans lequel pénètrent les filets, présente un maximum de surface convexe, en forme de vallées (10) entre les flancs de filets (11) pour faciliter, à ce niveau, l'ostéointégration par ostéogénèse de contact et remodelage sous contrainte, le relief de vissage étant agencé pour exercer un effet de coupe autotaraudant lors du vissage et un effet de compression de l'os spongieux.

7. Implant selon la revendication 6 **caractérisé en ce que**, dans un pas de filetage, la proportion de la largeur de filet, au niveau de la vallée, par rapport au pas, est comprise entre 0,2 et 0,5.

8. Implant selon l'une des revendications 1 à 7, **caractérisé en ce que** la section des filets est asymétrique dans un plan diamétral, avec un angle plus faible de l'ordre de 5 à 10° du côté polaire (7) du filet et un angle supérieur de l'ordre de 15 à 20° du côté équatorial (8), afin d'assurer un bon effet de compression, lors de la mise en contrainte de l'os recevant le filetage.

9. Implant selon l'une des revendications 1 à 8 **caractérisé en ce que** les crêtes de filets (11) sont détalonnées, avec un bord d'attaque radialement plus élevé que le reste de la crête, dont la hauteur radiale décroît vers l'arrière du filet.

10. Implant selon l'une des revendications 1 à 9 **caractérisé en ce que** le bord d'attaque est lui-même incliné en étant formé par une passe de fraisage fortement inclinées en biais par rapport à l'inclinaison du filetage lui-même, l'arête d'attaque (12) étant elle-même orientée agressivement vers l'avant par rapport à la radiale.

11. Implant selon l'une quelconque des revendications 1 à 10, **caractérisé en ce que** le pas de filetage est régulier afin de créer un seul sillon osseux, dans lequel pénètrent des filets successifs lors de la rotation de vissage.

12. Implant selon l'une des revendications 1 à 11 **caractérisé en ce que** le moyen de vissage possède un filetage formé de zones de filets (5) séparées par des gorges inclinées (6) déterminant les arêtes de coupe.

13. Implant selon l'une des revendications 1 à 12 **caractérisé en ce que** le moyen de vissage possède un filetage sphérique de pas constant.

14. Procédé de fabrication des cupules d'implant selon l'une des revendications 1 à 13 dans lequel on réalise une cupule, ayant un filetage dans sa zone équatoriale, on effectue un traitement de la surface externe de la cupule pour la rendre rugueuse et l'on met en place un revêtement de matériau ostéointégrateur sur ladite surface de cupule, **caractérisé en ce que** l'on mène l'étape de revêtement de matériau ostéointégrateur, de façon à réaliser un dépôt épais sur les surfaces convexes de la cupule, y compris les vallées séparant les filets voisins et en diminuant ou supprimant le revêtement par ledit matériau sur les flancs et bords des filets.

15. Procédé selon la revendication 14 **caractérisé en ce que** l'on utilise une torche à plasma pour projeter et fixer sur la surface rugueuse de la cupule le matériau destiné à former ledit revêtement et on réduit l'épaisseur du dépôt formant le revêtement sur les filets par modification temporaire de l'angle d'inclinaison de la torche et/ou par modification de la vitesse de défilement relative entre la torche et la cupule, et/ou par modification temporaire du débit de poudre d'hydroxyapatite de calcium.

16. Procédé de production de cupules d'implant **caractérisé par** les étapes suivantes :
- on détermine à l'avance, avec modification pendant le processus de production, le nombre de cupules revêtues ou non d'hydroxyapatite de calcium à fabriquer ;
- on réalise les cupules non revêtues et on les soumet à un traitement destiné à rendre leur surface rugueuse ;
- on détermine dans la production d'ensemble ainsi déterminée la proportion de cupules destinées à recevoir un revêtement de matériau ostéointégrateur ;
- on procède au revêtement des cupules destinées à être revêtues selon l'une des revendications 14 et 15;
- on conditionne et on stérilise l'ensemble des cupules pour être contenues dans des emballages stériles individuels, lesdits emballages présentant des marquages différents pour distinguer les cupules revêtues des cupules non revêtues.

## Claims

1. An acetabular implant comprising a cup to be screwed in and receiving an articular insert, said cup having on its periphery, and in particular in its tropico-equatorial zone (2), threading means intended to penetrate into the osseous material of the acetabulum during the screwing-in operation, said cup having a coating which facilitates osteo-integration, **characterised in that** the coating is thick on the convex portions (1,10) of the outer surface of the cup, including in the thread zones or valleys or hollows (10), left free in the threading means, whereas this coating is of lower thickness, or is even absent, on the reliefs or screw-threads (11).

2. An implant according to Claim 1, **characterised in that** the thickness of the thick coating is 100 to 200 microns.

3. An implant according to Claim 2, **characterised in that** the thickness of the coating is of the order of 150 ± 35 microns.

4. An implant according to any one of Claims 1 to 3, **characterised in that** the thread reliefs have a coating of the order of 50 ± 35 microns.

5. An implant according to any one of Claims 1 to 3, **characterised in that** the thread reliefs (11) do not have a coating and have a rough surface.

6. An implant according to any one of Claims 1 to 5, **characterised in that** the threading means, which is arranged so as to traumatise as little as possible the osseous acetabular site into which the threads penetrate, has a maximum convex surface in the form of valleys (10) between the thread flanks (11) so as to facilitate, at this level, osteointegration by contact osteogenesis and remodelling under pressure, the thread relief being designed to exert a self-tapping cutting effect during the screwing-in operation and a compression effect on the spongy bone.

7. An implant according to Claim 6, **characterised in that**, in one thread pitch, the proportion of the thread width, at the level of the valley, in relation to the pitch is between 0.2 and 0.5.

8. An implant according to any one of Claims 1 to 7, **characterised in that** the section of the threads is asymmetrical in a diametral plane, with a smaller angle of the order of 5 to 10° on the polar side (7) of the thread and a larger angle of the order of 15 to 20° on the equatorial side (8), so as to ensure a good compression effect when the bone receiving the screw-thread is placed under pressure.

9. An implant according to any one of Claims 1 to 8, **characterised in that** the crests of the threads (11) are backed off, with a leading edge which is radially higher than the remainder of the crest, the radial height of which decreases towards the rear of the thread.

10. An implant according to any one of Claims 1 to 9, **characterised in that** the leading edge is itself inclined by being formed by a milling pass, at a substantial inclination and obliquely in relation to the inclination of the screw-thread itself, the leading edge (12) itself being considerably oriented towards the front in relation to the radial.

11. An implant according to any one of Claims 1 to 10, **characterised in that** the thread pitch is regular so as to create a single osseous groove, into which successive threads penetrate during the screwing-in rotation.

12. An implant according to any one of Claims 1 to 11, **characterised in that** the threading means has a screw-thread in the form of thread zones (5) separated by inclined grooves (6) defining the cutting edges.

13. An implant according to any one of Claims 1 to 12, **characterised in that** the threading means has a constant-pitch spherical screw-thread.

14. A process for the manufacture of implant cups according to any one of Claims 1 to 13, in which a cup is formed which has a screw-thread in its equatorial zone, the outer surface of the cup undergoes a treatment to make it rough and a coating of osteointegrating material is applied to said cup surface, **characterised in that** the step of applying the coating of osteointegrating material is carried out so as to provide a thick deposit on the convex surfaces of the cup, including the valleys separating the adjacent threads, and decreasing or omitting the coating with said material on the flanks and edges of the threads.

15. A process according to Claim 14, **characterised in that** a plasma torch is used to project and fix on the rough surface of the cup the material intended to form said coating and the thickness of the deposit forming the coating is reduced on the threads by temporarily modifying the angle of inclination of the torch and/or by modifying the rate of relative displacement between the torch and the cup, and/or by temporarily modifying the flow rate of calcium hydroxyapatite powder.

16. A process for producing implant cups, **characterised by** the following steps:
- predetermining, with modification during the production process, the number of cups which are coated or not coated with calcium hydroxyapatite to be produced;
- producing the uncoated cups and subjecting them to a treatment intended to make their surface rough;
- determining in the overall production thus determined the proportion of cups intended to receive a coating of osteointegrating material;
- carrying out the coating of the cups to be coated in accordance with one of Claims 14 and 15;
- packing and sterilising all the cups so that they are contained in individual sterile packagings, said packagings bearing different markings so as to distinguish the coated and uncoated cups.

## Patentansprüche

1. Hüftpfannenimplantat mit einer zu verschraubenden Pfanne, welche einen Gelenkeinsatz aufnimmt, wobei die Pfanne an ihrem Umfang, und insbesondere in ihrem tropisch-äquatorialen Bereich (2) Verschraubungsvorrichtungen aufweist, welche dafür bestimmt sind, während der Verschraubung in das Knochenmaterial des Pfannendaches einzudringen, wobei die Pfanne eine die Knochenintegration erleichternde Beschichtung trägt, **dadurch gekennzeichnet, dass** die Beschichtung auf den konvexen Bereichen (1, 10) der Außenfläche der Pfanne, einschließlich in den Gewindebereichen oder -tälern oder -lücken (10), die in den Verschraubungsvorrichtungen frei gelassen sind, dick ausgebildet ist, wohingegen diese Beschichtung auf den Verschraubungserhöhungen oder - gewinden (11) eine geringere Dicke aufweist oder sogar ganz fehlt.

2. Implantat gemäß Anspruch 1, **dadurch gekennzeichnet, dass** die Dicke der dicken Beschichtung zwischen 100 und 200 Mikrometern beträgt.

3. Implantat gemäß Anspruch 2, **dadurch gekennzeichnet, dass** die Beschichtungsdicke in der Größenordnung von 150 ± 35 Mikrometern liegt.

4. Implantat gemäß einem der Ansprüche 1 bis 3, **dadurch gekennzeichnet, dass** die Verschraubungserhöhungen eine Beschichtung in der Größenordnung von 50 ± 30 Mikrometern aufweisen.

5. Implantat gemäß einem der Ansprüche 1 bis 3, **dadurch gekennzeichnet, dass** die Verschraubungserhöhungen (11) keine Beschichtung aufweisen und eine raue Oberfläche besitzen.

6. Implantat gemäß einem der Ansprüche 1 bis 5" **dadurch gekennzeichnet, dass** die Verschraubungsvorrichtung, die dafür eingerichtet ist, die Knochenstelle der Hüftpfanne, in welche die Gewinde eindringen, so wenig wie möglich zu traumatisieren, eine maximale konvexe Oberfläche in Form von Tälern (10) zwischen den Gewindeflanken (11) aufweist, um in diesem Bereich die Knochenintegration durch Kontaktosteogenese und Remodellierung unter Spannung zu erleichtern, wobei die Verschraubungserhöhung dafür eingerichtet ist, während der Verschraubung eine selbstschneidende Schneidwirkung und eine Komprimierungswirkung auf den schwammigen Knochen auszuüben.

7. Implantat gemäß Anspruch 6, **dadurch gekennzeichnet, dass** in einer Gewindesteigung das Verhältnis der Gewindebreite im Bereich des Tals zu der Steigung zwischen 0,2 und 0,5 liegt.

8. Implantat gemäß einem der Ansprüche 1 bis 7, **dadurch gekennzeichnet, dass** der Querschnitt der Gewinde in einer diametralen Ebene asymmetrisch ausgebildet ist, mit einem kleineren Winkel in der Größenordnung von 5 bis 10° auf der polaren Seite (7) des Gewindes und einem größeren Winkel in der Größenordnung von 15 bis 20° auf der äquatorialen Seite (8), um eine gute Komprimierungswirkung sicherzustellen während der die Gewinde aufnehmende Knochen unter Spannung gesetzt wird.

9. Implantat gemäß einem der Ansprüche 1 bis 8, **dadurch gekennzeichnet, dass** die Gewindekronen (11) hinterschnitten sind, mit einer radial höheren Vorderkante als der Rest der Krone, deren radiale Höhe zum hinteren Bereich des Gewindes hin abnimmt.

10. Implantat gemäß einem der Ansprüche 1 bis 9, **dadurch gekennzeichnet, dass** die Vorderkante selbst geneigt ist, indem sie von einer Fräskante gebildet ist, welche bezüglich der Neigung des Gewindes selbst stark schräg geneigt ist, wobei die Vorderkante (12) selbst bezüglich der Radialen aggressiv nach vorne ausgerichtet ist.

11. Implantat gemäß irgendeinem der Ansprüche 1 bis 10, **dadurch gekennzeichnet, dass** die Gewindesteigung regelmäßig ausgebildet ist, um eine einzige Knochenfurche zu schaffen, in welche aufeinander folgende Gewinde während der Einschraubdrehung eindringen.

12. Implantat gemäß einem der Ansprüche 1 bis 11, **dadurch gekennzeichnet, dass** die Verschraubungsvorrichtung ein Gewinde besitzt, welches aus Gewindebereichen (5) gebildet ist, die durch die Schneidkanten bestimmende, geneigte Rillen (6) voneinander getrennt sind.

13. Implantat gemäß einem der Ansprüche 1 bis 12, **dadurch gekennzeichnet, dass** die Verschraubungsvorrichtung ein kugelförmiges Gewinde mit konstanter Steigung besitzt.

14. Verfahren zur Herstellung von Implantatpfannen gemäß einem der Ansprüche 1 bis 13, bei welchem eine Pfanne hergestellt wird, welche in ihrem äquatorialen Bereich ein Gewinde aufweist, eine Behandlung der Außenfläche der Pfanne vorgenommen wird, um sie aufzurauen und eine Beschichtung aus Knochen integrierendem Material auf die Pfannenoberfläche aufgebracht wird, **dadurch gekennzeichnet, dass** der Schritt des Aufbringens der Beschichtung aus Knochen integrierendem Material derart durchgeführt wird, dass auf die konvexen Oberflächen der Pfanne, einschließlich der Täler, welche die benachbarten Gewinde voneinander trennen, eine dicke Schicht aufgebracht wird, und dass die Beschichtung aus diesem Material auf den Flanken und Kanten der Gewinde verringert oder weggelassen wird.

15. Verfahren gemäß Anspruch 14, **dadurch gekennzeichnet, dass** eine Plasmafackel verwendet wird, um auf der rauen Oberfläche der Pfanne das Material aufzubringen und zu befestigen, das dafür bestimmt ist, die Beschichtung zu bilden, und dass die Dicke der auf den Gewinden die Beschichtung bildenden Ablagerung durch zeitweise Veränderung des Neigungswinkels der Fackel und/oder durch zeitweise Veränderung der relativen Vorbeilaufgeschwindigkeit zwischen der Fackel und Pfanne und/oder durch zeitweise Veränderung des Durchsatzes an Calciumhydroxyapatitpulver verringert wird.

16. Verfahren zur Herstellung von Implantatpfannen, **gekennzeichnet durch** die folgenden Schritte:
- im voraus wird die Anzahl an herzustellenden mit Calciumhydroxyapatit beschichteten oder nicht beschichteten Pfannen festgelegt, und zwar mit Modifizierung während des Herstellungsvorgangs;
- die nicht beschichteten Pfannen werden hergestellt und einer Behandlung unterzogen, die dafür bestimmt ist, ihre Oberfläche aufzurauen;
- in der so festgelegten Gesamtproduktion wird der Anteil an Pfannen bestimmt, die dafür bestimmt sind, eine Beschichtung aus Knochen integrierendem Material aufzunehmen;
- die Beschichtung dieser Pfanne wird gemäß einem der Ansprüche 14 und 15 vorgenommen;
- die Gesamtheit an Pfannen wird verpackt und sterilisiert, um in sterilen Einzelverpackungen aufgenommen zu werden, wobei die Verpackungen unterschiedliche Kennzeichnungen aufweisen, um die beschichteten Pfannen von den nicht beschichteten Pfannen zu unterscheiden.
